# EUROPEAN PATENT SPECIFICATION

(11) **EP 4 042 167 B1**
(45) Date of publication and mention of the grant of the patent: **09.04.2025**
(21) Application number: 20793213.8
(22) Date of filing: 28.09.2020
(51) Int. Cl.: G01N 33/68, C07K 16/24

(54) **COMPOSITIONS AND METHODS FOR AN IL-17 TARGET ENGAGEMENT ASSAY WITH LARGE MOLECULE MODULATORS**
ZUSAMMENSETZUNGEN UND VERFAHREN FÜR EINEN IL-17-ZIELEINGRIFFS-ASSAY MIT GROSSEN MOLEKÜLEN MODULATOREN
COMPOSITIONS ET MÉTHODES POUR UN DOSAGE D'ENTRÉE EN PRISE CIBLE D'IL-17 AVEC DES MODULATEURS À GRANDES MOLÉCULES

(30) Priority: 30.09.2019 US 201962908195 P; 12.01.2020 US 202062960031 P; 14.09.2020 US 202063077992 P
(43) Date of publication of application: 17.08.2022
(73) Proprietor: Janssen Pharmaceutica NV, 2340 Beerse (BE)
(72) Inventor: LEUNG, Wai-Ping, San Diego, CA 92121 (US); BLEVITT, Jonathan, San Diego, CA 92121 (US); DE LEON-TABALDO, Aimee, Rose, San Diego, CA 92121 (US); XUE, Xiaohua, San Diego, CA 92121 (US)
(74) Representative: Carpmaels & Ransford LLP
(86) International application number: PCT/US2020/053112
(87) International publication number: WO 2021/067195

(56) References cited:
- ANONYMOUS: "CENTER FOR DRUG EVALUATION AND RESEARCH CLINICAL PHARMACOLOGY INDIVIDUAL STUDY SUMMARY", 24 October 2013 (2013-10-24), XP055408601, Retrieved from the Internet <URL:https://www.accessdata.fda.gov/drugsatfda_docs/nda/2015/125504Orig1s000ClinPharmR.pdf> [retrieved on 20170921]
- PENG KUN ET AL: "Measurement of IL-17AA and IL-17FF as Pharmacodynamic Biomarkers to Demonstrate Target Engagement in the Phase I Study of MCAF5352A", THE AAPS JOURNAL, SPRINGER INTERNATIONAL PUBLISHING, CHAM, vol. 21, no. 1, 13 December 2018 (2018-12-13), pages 1 - 9, XP036665454, DOI: 10.1208/S12248-018-0280-Z

## Description

### BACKGROUND

Interleukin-17A (IL-17 or IL-17A) is a cytokine secreted by activated Th17 cells, CD8+ T cells, γδ T cells, and NK cells in response to cytokines such as IL-23 and TGF-β. IL-17 regulates production of mediators such as antimicrobial peptides, proinflammatory cytokines, and chemokines from multiple cell types including epithelia cells, fibroblasts, and synoviocytes that are involved in the recruitment of neutrophils and in the pathology of tissue damage in inflammation or in host defense. IL-17 also synergizes with other cytokines, such as TNF-α and IL-Iβ to potentiate a pro-inflammatory environment.

Due to its involvement in immune regulatory functions, inhibitors of IL-17 are being investigated as possible treatments for various autoimmune diseases. Anti-IL-17 monoclonal antibodies (mAbs) are validated clinically for the treatment of psoriasis, psoriatic arthritis, ankylosing spondylitis and have demonstrated proof of concept for the treatment of multiple sclerosis.

Despite the clinical utility of anti-IL-17 mAbs, hereafter referred to as large molecule (LM) modulator(s) of IL-17, target engagement assays for LM modulators of IL-17 remains lacking. Specifically, in studies involving LM modulators, commercially available assays in the art can usually only measure the amount of free IL-17, but not LM-bound IL-17, in a biological sample comprising IL-17 and the LM modulator(s). The "Center for drug evaluation and research clinical pharmacology individual study summary" (https://www.accessdata.fda.gov/drugsatfda_docs/nda/2015/125504Orig1s000 ClinPharmR.pdf; retrieved on 2017-09-21) describes individual study summaries for clinical pharmacology and clinical studies relating to secukinumab. Additionally, even though Peng *et al.* developed an assay that measured target engagement specifically for one anti-IL-17 antibody, MCAF5352A, this method demonstrates utility with a single antibody. *See, i.e.,* Peng et al., Measurement of IL-17AA and IL-17FF as Pharmacodynamic Biomarkers to Demonstrate Target Engagement in the Phase I Study of MCAF53 52A, The AAPS Journal (2019) 21: 9). Accordingly, information on levels of IL-17 target engagement by multiple LM modulators is lacking.

Moreover, although disease related biomarkers have been used to monitor anti-IL-17 therapeutic effects in patients, no biomarkers have been developed to monitor anti-IL-17 effects in healthy subjects who are often included at early stages of clinical evaluation. As a result, a measurement that can demonstrate the target engagement of an IL-17 LM modulator with IL-17 in a subject (*e.g.,* a human) is important to progressing the LM modulators through clinical studies. There is also an additional need for method(s) to assess IL-17 engagement by LM modulators in a sample to study clinically relevant parameters such as pharmacokinetic (PK) and pharmacodynamic (PD) information, which in turn, may aid in the determination of an optimal dosage for a safe and efficacious therapy. Thus, there remains a need for a robust immunoassay to assess the amount of total IL-17 (*e.g.,* IL-17 that are bound and unbound to LM modulators) and to further provide target engagement information in samples involving a larger number of LM modulators.

### BRIEF SUMMARY

In a general aspect, the application relates to an immunoassay that can detect the total IL-17 (that is, both free IL-17 and modulator-bound IL-17), in a sample comprising IL-17 and LM modulator, such that the target engagement of LM modulators can be evaluated by subtracting the level of free IL-17 from the level of total IL-17.

Provided herein is a method of determining a total amount of IL-17 (free IL-17 and LM modulator-bound IL-17) in a sample comprising an IL-17 and a large molecule (LM) modulator of IL-17, the method comprising:
i. contacting the sample with a capture antibody to form a mixture comprising a first complex comprising the capture antibody and the IL-17 unbound to the LM modulator, and a second complex comprising the capture antibody and the IL-17 bound to the LM modulator;
ii. contacting the mixture from step i) with a detection agent to thereby form a third complex comprising the capture antibody, the IL-17 unbound to the LM modulator and the detection agent, and a fourth complex comprising the capture antibody, the IL-17 bound to the LM modulator and the detection agent; and
iii. determining the amount of total IL-17 by measuring the amount of the detection agent in the third and fourth complexes,
wherein the capture antibody is anti-IL-17 antibody AF-317 and the detection agent is mAb4538, the large molecule (LM) modulator is selected from the group consisting of secukinumab, mAb7024, and mAb732 and the capture antibody is capable of forming a complex comprising the capture antibody and the IL-17 bound to any of secukinumab, mAb7024, and mAb732.

In a further embodiment of the method, a wash step is performed before contacting the first mixture from step i) with the detection agent in step (ii).

In a yet further embodiment of the method, LM modulator issecukinumab or mAb7024.

In a yet further embodiment of the method, the detection agent is labeled with a detectable label.

In a yet further embodiment of the method, the detectable label is an enzyme or biotin.

In a yet further embodiment of the method, the sample is a biological sample obtained from a subject under an *ex vivo* or *in vivo* treatment with the LM modulator.

In a yet further embodiment of the method, the subject is a mammal, preferably human.

In a yet further embodiment of the method, the biological sample is selected from the group consisting of a sample prepared from cells, tissues, or a serum, plasma or another biological fluid sample.

In a yet further embodiment of the method, the immunoassay is an enzyme-linked immunosorbent assay (ELISA).

Further provided herein is a kit for determining an amount of total IL-17 in a sample comprising an IL-17 and a LM modulator of IL-17, the kit comprising:
i. a capture antibody, wherein upon contact with the sample, the capture antibody forms a first complex comprising the capture antibody and the IL-17 unbound to the LM modulator, and a second complex comprising the capture antibody and the IL-17 bound to the LM modulator; and
ii. a detection agent, wherein upon contact with the first complex and the second complex, the detection agent, forms a third complex comprising the capture antibody, the IL-17 unbound to the LM modulator, and the detection agent, and a fourth complex comprising the capture antibody, the IL-17 bound to the LM modulator, and the detection agent,
wherein, the capture antibody is anti-IL-17 antibody AF-317 and the detection agent is mAb4538, the large molecule (LM) modulator is selected from the group consisting of secukinumab, mAb7024, and mAb732 and the capture antibody is capable of forming a complex comprising the capture antibody and the IL-17 bound to any of secukinumab, mAb7024, and mAb732.

In a further embodiment of the kit, the detection agent is labeled with a detectable label, more preferably, the label is an enzyme or biotin.

In a yet further embodiment, the kit is for conducting ELISA.

Other aspects, features and advantages of the invention will be apparent from the following disclosure, including the detailed description of the invention and its preferred embodiments and the appended claims.

### BRIEF DESCRIPTION OF THE DRAWINGS

The foregoing summary, as well as the following detailed description of preferred embodiments of the present application, will be better understood when read in conjunction with the appended drawings. It should be understood, however, that the application is not limited to the precise embodiments shown in the drawings.

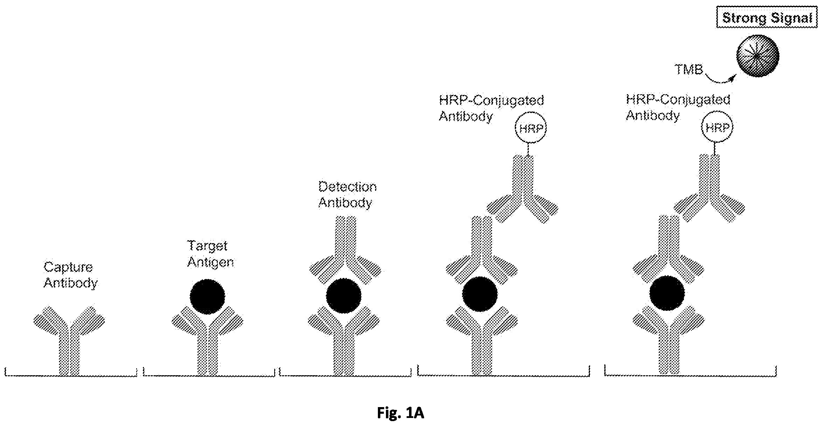

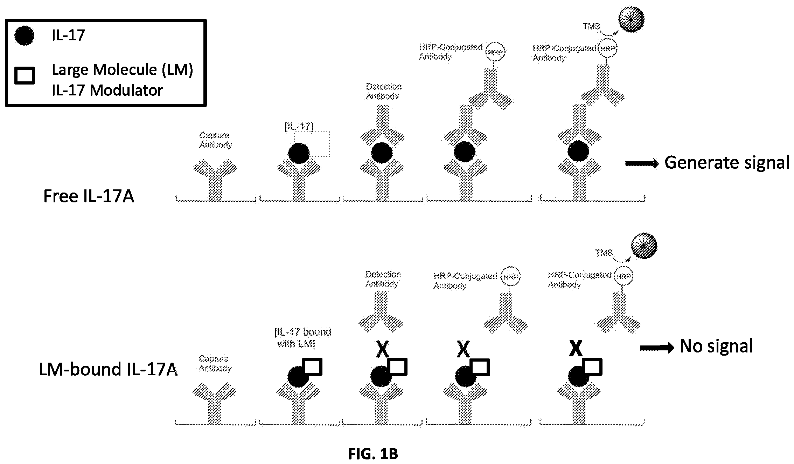

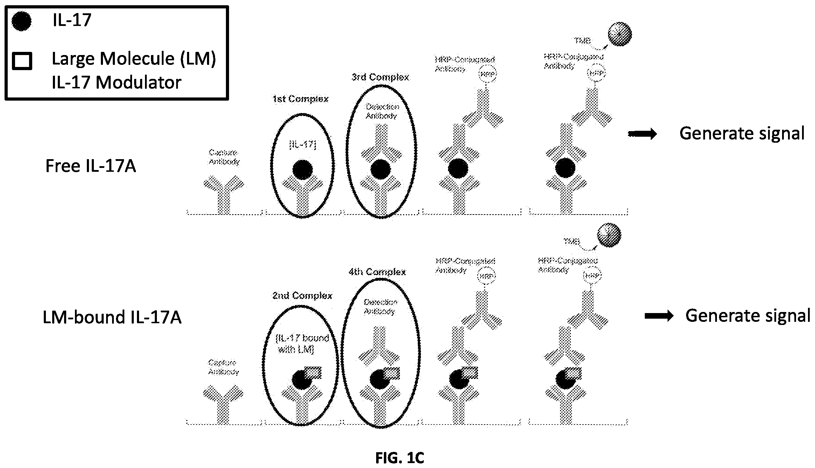

**FIGS. 1A-1C** show a schematic representation of an IL-17 detection assay: FIG. 1A - conventional immunoassay to detect target antigen (e.g., IL-17) using a capture antibody/detection agent pair; FIG. 1B - immunoassay to detect free IL-17 (i.e., IL-17 that are not bound to modulators), in the presence of LM modulators, using commercially available capture antibody/detection agent pair; and FIG. 1C - immunoassay to detect total IL-17 (including both free IL-17 and modulator-bound IL-17), in the presence of LM modulators, using inventive capture antibody/detection agent pairs.
**FIG. 2** shows detection of free IL-17 using DuoSet ELISA DY317 (R&D Systems, Inc.) with LM modulator of IL-17 (secukinumab, at 10, 1, 0.1, or 0.01 µg/ml) or without LM modulator.
**FIG. 3** shows detection of total IL-17 using an anti-IL-17 antibody AF-317 (cat# AF-317-NA from R&D systems) as capture antibody and mAb4538 as detection agent in samples with LM modulator of IL-17 (secukinumab, at 10, 1, 0.1, 0.01, or 0.001 µg/ml) or without LM modulator.
**FIG. 4A** shows detection of free IL-17 using DuoSet ELISA kit DY317 in samples with LM modulator of IL-17 (secukinumab, mAb7024, or mAb732) or without LM modulator.
**FIG. 4B** shows detection of total IL-17 using an anti-IL-17 antibody AF-317 (cat# AF-317-NA, R&D systems) as capture antibody and mAb4538 as detection agent in samples with LM modulator of IL-17 (secukinumab, mAb7024, or mAb732) or without LM modulator.

### DETAILED DESCRIPTION

Various publications, articles and patents are cited or described in the background and throughout the specification. Discussion of documents, acts, materials, devices, articles or the like which has been included in the present specification is for the purpose of providing context for the invention. Such discussion is not an admission that any or all of these matters form part of the prior art with respect to any inventions disclosed or claimed.

Unless defined otherwise, all technical and scientific terms used herein have the same meaning as commonly understood to one of ordinary skill in the art to which this invention pertains. Otherwise, certain terms used herein have the meanings as set forth in the specification.

It must be noted that as used herein and in the appended claims, the singular forms "a," "an," and "the" include plural reference unless the context clearly dictates otherwise.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim. Any of the aforementioned terms of "comprising", "containing", "including", and "having", whenever used herein in the context of an aspect or embodiment of the application can be replaced with the term "consisting of" or "consisting essentially of" to vary scopes of the disclosure.

As used herein, the conjunctive term "and/or" between multiple recited elements is understood as encompassing both individual and combined options. For instance, where two elements are conjoined by "and/or," a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Unless otherwise stated, any numerical value, such as a concentration or a concentration range described herein, are to be understood as being modified in all instances by the term "about." Thus, a numerical value typically includes ± 10% of the recited value. For example, a concentration of 1 mg/mL includes 0.9 mg/mL to 1.1 mg/mL. Likewise, a concentration range of 1 mg/mL to 10 mg/mL includes 0.9 mg/mL to 11 mg/mL. As used herein, the use of a numerical range expressly includes all possible subranges, all individual numerical values within that range, including integers within such ranges and fractions of the values unless the context clearly indicates otherwise.

The phrases "percent (%) sequence identity" or "% identity" or "% identical to" when used with reference to an amino acid sequence describe the number of matches ("hits") of identical amino acids of two or more aligned amino acid sequences as compared to the number of amino acid residues making up the overall length of the amino acid sequences. In other terms, using an alignment, for two or more sequences the percentage of amino acid residues that are the same (e.g. 90%, 91%, 92%, 93%, 94%, 95%, 97%, 98%, 99%, or 100% identity over the full-length of the amino acid sequences) may be determined, when the sequences are compared and aligned for maximum correspondence as measured using a sequence comparison algorithm as known in the art, or when manually aligned and visually inspected. The sequences which are compared to determine sequence identity may thus differ by substitution(s), addition(s) or deletion(s) of amino acids. Suitable programs for aligning protein sequences are known to the skilled person. The percentage sequence identity of protein sequences can, for example, be determined with programs such as CLUSTALW, Clustal Omega, FASTA or BLAST, e.g. using the NCBI BLAST algorithm (Altschul SF, et al (1997), Nucleic Acids Res. 25:3389-3402).

As used herein, "subject" means any animal, preferably a mammal, most preferably a human, who will be or has been treated by a method according to an embodiment of the application. The term "mammal" as used herein, encompasses any mammal. Examples of mammals include, but are not limited to, cows, horses, sheep, pigs, cats, dogs, mice, rats, rabbits, guinea pigs, non-human primates (NHPs) such as monkeys or apes, humans, etc., more preferably a human.

In an attempt to help the reader of the application, the description has been separated in various paragraphs or sections, or the reader is directed to various embodiments of the application. These separations should not be considered as disconnecting the substance of a paragraph or section or embodiments from the substance of another paragraph or section or embodiments. To the contrary, one skilled in the art will understand that the description has broad application and encompasses all the combinations of the various sections, paragraphs and sentences that can be contemplated. The discussion of any embodiment is meant only to be exemplary and is not intended to suggest that the scope of the disclosure, including the claims, is limited to these examples.

As used herein, "IL-17" or "IL-17A" refers to interleukin 17A. It is also named IL17, CTLA8, CTLA-8. Interleukin 17A is a pro-inflammatory cytokine. This cytokine is produced by a group of T helper cell known as T helper 17 cell in response to their stimulation with IL-23. The protein encoded by IL17A is a founding member of IL-17 family, including IL17A, IL-17B, IL-17C, IL-17D, IL-17E and IL-17F. IL-17E is also known as IL-25. All members of the IL-17 family have a similar protein structure. IL-17 regulates the activities of NF-kappaB and mitogen-activated protein kinases. It can stimulate the expression of IL6 and cyclooxygenase-2 (PTGS2/COX-2), as well as enhance the production of nitric oxide (NO). High levels of IL17 are associated with several chronic inflammatory diseases including rheumatoid arthritis, psoriasis and multiple sclerosis. An "IL-17" includes an IL-17A from any animal species, as well as a recombinant product of IL-17A. IL-17 could be in homodimer of IL-17A or in heterodimer of IL-17A and IL-17F. An exemplary amino acid sequence of human IL-17 is represented in GenBank Accession No. NP_002181.1, which can be encoded by a nucleic acid sequence such as that of GenBank Accession No. NM_002190.3.

The term "modulator" as used herein refers to any agents or molecules that can bind to IL-17, including small molecule compounds and large molecules such as antibodies.

The terms "small molecule modulator(s)" and/or "SM modulator(s)" and/or "SM modulator(s) of IL-17" are used interchangeably herein and refer to any small molecule compound that can bind to IL-17 and has a molecular weight ranging from about 100 g/mol to about 1500 g/mol.

The terms "large molecule modulator(s)" and/or "LM modulator(s)" and/or "LM modulator(s) of IL-17" are used interchangeably herein and refer to any large molecule compound (e.g., antibodies, proteins, etc.) that can bind to IL-17 and has a molecular weight of above about 1500 daltons, or preferably has a molecular weight ranging from about 2,000 daltons to about 250,000 daltons, or from about 5000 daltons to about 160,000 daltons, or from about 150,000 daltons to about 250,000 daltons. The dalton (symbol Da) is used herein as a unit of molar mass, with the definition 1 Da = 1 g/mol.

The term "free IL-17" or "unbound IL-17" as used herein refers IL-17 that are not bound to any modulators in a biological sample.

The terms "sample" and "biological sample" are used interchangeably herein and encompass a variety of sample types obtained or derived from an organism and can be used in a diagnostic or monitoring assay. The terms encompass blood and other liquid samples of biological origin, solid tissue samples, such as a biopsy specimen or tissue cultures or cells derived therefrom and the progeny thereof. The terms encompass samples that have been manipulated in any way after their procurement, such as by treatment with reagents, solubilization, or enrichment for certain components. The terms encompass clinical samples, and also include cells in cell culture, cell supernatants, cell lysates, serum, plasma, biological fluids, and tissue samples.

As used herein, the term "antibody" is used in a broad sense and includes immunoglobulin or antibody molecules including human, humanized, composite and chimeric antibodies and antibody fragments that are monoclonal or polyclonal. In general, antibodies are proteins or peptide chains that exhibit binding specificity to a specific antigen. The antibody can be derived from any species and can be IgM, IgG (e.g. IgGl, IgG2, IgG3, or IgG4), IgD, IgA, or IgE, for example.

As used herein, the term "antigen-binding fragment" refers to an antibody fragment such as, for example, a diabody, a Fab, a Fab', a F(ab')2, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), a single domain antibody (sdab) an scFv dimer (bivalent diabody), a multispecific antibody formed from a portion of an antibody comprising one or more CDRs, a camelized single domain antibody, a nanobody, a domain antibody, a bivalent domain antibody, or any other antibody fragment that binds to an antigen but does not comprise a complete antibody structure, or any peptide comprising an antigen-binding fragment.

The term "monoclonal antibody" as used herein refers to a preparation of antibodies of single molecular composition.

The term "polyclonal antibody" as used herein refers to a composition of different antibody molecules which is capable of binding to or reacting with several different specific antigenic determinants on the same or on different antigens. The variability in antigen specificity of a polyclonal antibody is located in the variable regions of the individual antibodies constituting the polyclonal antibody, in particular in the complementarity determining regions CDRl, CDR2 and CDR3 regions.

The term "capture antibody" as used herein refers to an antibody that specifically binds to IL-17 regardless of whether the IL-17 is bound to a modulator or not. Such capture antibodies, can be commercially available or investigational agents. Capture antibodies can be provided in solution or pre-coated to a surface.

The term "detection agent" as used herein refers to an agent that specifically binds to either an IL-17 unbound to a modulator or the IL-17 bound to the modulator, or both. A "detection agent," such as a detecting antibody, or a detecting soluble receptor, can be used to detect either an IL-17 unbound to a modulator or the IL-17 bound to the modulator. Detection agents, such as detection antibodies, can be commercially available or investigational. The detection agent can be detected by any method known in the art, such as using a radioactive or fluorescent tag, or by binding directly or indirectly to an enzyme or biotin. Preferably, the detection agent, such as detection antibody, is labeled with a detectable label, such as an enzyme or biotin.

The enzyme can convert certain substrates to detectable products. For example, the enzyme can be one of the following enzymes commonly used in enzyme-linked immunosorbent assays (ELISA):
- Horseradish Peroxide (HRP), often used for conjugation with a protein, turns o-phenylenediamine dihydrochloride (OPD) into an amber product;
- HRP turns 3,3',5,5'-tetramethylbenzidine (TMB) into a blue product which becomes yellow in the presence of sulfuric or phosphoric acid;
- HRP turns 2,2'-Azinobis [3-ethylbenzothiazoline-6-sulfonic acid]-diammonium salt (ABTS) into a green product; and
- Alkaline phosphatase turns p-nitrophenyl phosphate (PNPP) into a yellow product.

Biotin is a water-soluble B vitamin, also called vitamin B7. Biotin can bind to Avidin, Streptavidin and NeutrAvidin with considerable affinity. These proteins (Avidin, Streptavidin and NeutrAvidin) can be further conjugated to the aforementioned detectable enzymes to produce signals.

The term "specifically binding" refers to binding between two agents, for example, an antigen and an antibody, characterized by the ability of one agent (e.g., antigen) to associate with another agent (e.g., antibody) even in the presence of many other diverse agents, i.e., to show preferential binding of one agent for another in a heterogeneous mixture of agents. For example, an antibody that "specifically binds to" an antigen refers to an antibody or an antigen binding fragment thereof that binds to the antigen with a KD of 1×10⁻⁷ M or less, preferably 1×10⁻⁸ M or less, more preferably 5×10⁻⁹ M or less, 1×10⁻⁹ M or less, 5×10⁻¹⁰ M or less, or 1×10⁻¹⁰ M or less. KD is the equilibrium dissociation constant, a calculated ratio of K_{off}/Kₒₙ, between the antibody and its antigen. For example, the KD of an antibody can be determined by using surface plasmon resonance, such as by using a biosensor system, e.g., a Biacore^{®} system, or by using bio-layer interferometry technology, such as an Octet RED96 system. The smaller the value of the KD of an antibody, the higher affinity that the antibody binds to a target antigen.

The term "kit" as used herein refers to a combination of reagents and other materials. It is contemplated that the kit may include reagents such as buffering agents, protein stabilizing reagents, signal producing systems (e.g., florescence signal generating systems), antibodies, control proteins, as well as testing containers (e.g., microtiter plates, etc.). It is not intended that the term "kit" be limited to a particular combination of reagents and/or other materials. In one embodiment, the kit further comprises instructions for using the reagents. The test kit may be packaged in any suitable manner, typically with the elements in a single container or various containers as necessary along with a sheet of instructions for carrying out the test. In some embodiments, the kits also preferably include a positive control sample. Kits may be produced in a variety of ways known in the art.

The present application relates generally to an IL-17 target engagement assay to measure the amount of total IL-17 (that is, IL-17 bound or not bound to an LM modulator(s)) in a sample comprising the IL-17 and an LM modulator(s).

In studies involving LM modulators, commercially available assays in the art can usually only measure the amount of free IL-17 in a biological sample comprising IL-17 and the LM modulator(s). As discussed previously, even though Peng *et al.* developed an assay that measured target engagement specifically for one anti-IL-17 antibody, MCAF5352A, the present application provides methods of determining the amount of total IL-17 (e.g., IL-17 that are bound and unbound to LM modulators), for a larger number of LM modulators. The method comprising:
i. contacting the sample with a capture antibody to form a mixture comprising a first complex comprising the capture antibody and the IL-17 unbound to the LM modulator, and a second complex comprising the capture antibody and the IL-17 bound to the LM modulator;
ii. contacting the mixture from step i) with a detection agent (preferably a detection antibody) to thereby form a third complex comprising the capture antibody, the IL-17 unbound to the LM modulator and the detection agent, and a fourth complex comprising the capture antibody, the IL-17 bound to the LM modulator and the detection agent; and
iii. determining the amount of total IL-17 by measuring the amount of the detection agent in the third and fourth complexes
wherein, the capture antibody is anti-IL-17 antibody AF-317 and the detection agent is mAb4538 and the large molecule (LM) modulator is selected from the group consisting of secukinumab, mAb7024, and mAb732 and the capture antibody is capable of forming a complex comprising the capture antibody and the IL-17 bound to any of secukinumab, mAb7024, and mAb732.

After contacting the sample with the capture antibody, the capture antibody specifically binds to IL-17 (both free IL-17 and those bound with LM modulators) in the sample to form the first and second complexes, respectively, in the mixture. In certain embodiments, the capture antibody is attached to a solid carrier and the mixture contains the first and second IL-17 complexes attached to the solid carrier together with the rest of the sample.

As used herein, the term "detection agent" refers to an agentwhich specifically binds to both the free IL-17 that are captured by the capture antibody and the modulator-bound IL-17 that are captured by the capture antibody. In particular, when contacting the detection agent with the first and second complexes, it forms a third complex comprising the capture antibody, the free IL-17, and the detection agent, and a fourth complex comprising the capture antibody, the modulator-bounded IL-17, and the detection agent.

Thus, the capture antibody and the detection agent are used in pairs to measure the amount of total IL-17 in a sample.

Detailed sequence information for antibodies of the disclosure is listed in Table 1.

**Table 1. Antibody Sequence**

| Antibody ID | Sequence |
|---|---|
| mAb4538 | LCDR1 - SEQ ID NO: 1 |
| | RASQNVWAFYLA |
| | LCDR2 - SEQ ID NO: 2 |
| | GASNRAT |
| | LCDR3 - SEQ ID NO: 3 |
| | QQYRSTLSLT |
| | HCDR1 - SEQ ID NO: 4 |
| | SSSAAWG |
| | HCDR2 - SEQ ID NO: 5 |
| | RISYRSKWYNDYAVSVKS |
| | HCDR3 - SEQ ID NO: 6 |
| | EVDSMYYSYFDI |
| | VL - SEQ ID NO: 7 |
| | EIVLTQSPATLSLSPGERATLSCRASQNVWAFYLAWYQQKPGQ APRLLIYGASNRATGIPARFSGSGSGTDFTLTISSLEPEDFAVYY CQQYRSTLSLTFGQGTKVEIK |
| | VH - SEQ ID NO: 8 |
| | QVQLQQSGPGLVKPSQTLSLTCAISGDSVSSSSAAWGWIRQSPS RGLEWLGRISYRSKWYNDYAVSVKSRITINPDTSKNQFSLQLNS VTPEDTAVYYCAREVDSMYYSYFDIWGQGTLVTVSS |
| | LC - SEQ ID NO: 9 |
| | EIVLTQSPATLSLSPGERATLSCRASQNVWAFYLAWYQQKPGQ APRLLIYGASNRATGIPARFSGSGSGTDFTLTISSLEPEDFAVYY CQQYRSTLSLTFGQGTKVEIKGQPKAAPSVTLFPPSSEELQANK |
| | ATLVCLISDFYPGAVTVAWKADSSPVKAGVETTTPSKQSNNKY AASSYLSLTPEQWKSHRSYSCQVTHEGSTVEKTVAPTECS |
| | HC - SEQ ID NO: 10 |
| | QVQLQQSGPGLVKPSQTLSLTCAISGDSVSSSSAAWGWIRQSPS RGLEWLGRISYRSKWYNDYAVSVKSRITINPDTSKNQFSLQLNS VTPEDTAVYYCAREVDSMYYSYFDIWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSRDELTKNQVSLTCLVKGFYPSDIAVEWESNG QPENNYKTTPPVLDSDGSFFLYSKLTVDKSRWQQGNVFSCSVM HEALHNHYTQKSLSLSPGK |
| mAb732 | LCDR1 - SEQ ID NO: 1 |
| | RASQNVWAFYLA |
| | LCDR2 - SEQ ID NO: 2 |
| | GASNRAT |
| | LCDR3 - SEQ ID NO: 11 |
| | TQYYSSPSLT |
| | HCDR1 - SEQ ID NO: 4 |
| | SSSAAWG |
| | HCDR2 - SEQ ID NO: 5 |
| | RISYRSKWYNDYAVSVKS |
| | HCDR3 - SEQ ID NO: 6 |
| | EVDSMYYSYFDI |
| | VL - SEQ ID NO: 12 |
| | EIVLTQSPATLSLSPGERATLSCRASQNVWAFYLAWYQQKPGQ APRLLIYGASNRATGIPARFSGSGSGTDFTLTISSLEPEDFAVYY CTQYYSSPSLTFGQGTKVEIK |
| | VH - SEQ ID NO: 8 |
| | QVQLQQSGPGLVKPSQTLSLTCAISGDSVSSSSAAWGWIRQSPS RGLEWLGRISYRSKWYNDYAVSVKSRITINPDTSKNQFSLQLNS VTPEDTAVYYCAREVDSMYYSYFDIWGQGTLVTVSS |
| | LC - SEQ ID NO: 22 |
| | EIVLTQSPATLSLSPGERATLSCRASQNVWAFYLAWYQQKPGQ APRLLIYGASNRATGIPARFSGSGSGTDFTLTISSLEPEDFAVYY CTQYYSSPSLTFGQGTKVEIKGQPKAAPSVTLFPPSSEELQANK ATLVCLISDFYPGAVTVAWKADSSPVKAGVETTTPSKQSNNKY AASSYLSLTPEQWKSHRSYSCQVTHEGSTVEKTVAPTECS |
| | HC - SEQ ID NO: 10 |
| | QVQLQQSGPGLVKPSQTLSLTCAISGDSVSSSSAAWGWIRQSPS RGLEWLGRISYRSKWYNDYAVSVKSRITINPDTSKNQFSLQLNS VTPEDTAVYYCAREVDSMYYSYFDIWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSRDELTKNQVSLTCLVKGFYPSDIAVEWESNG QPENNYKTTPPVLDSDGSFFLYSKLTVDKSRWQQGNVFSCSVM HEALHNHYTQKSLSLSPGK |
| mAb7024 | LCDR1 - SEQ ID NO: 13 |
| | RASQGISNWLN |
| | LCDR2 - SEQ ID NO: 14 |
| | AASSLQS |
| | LCDR3 - SEQ ID NO: 15 |
| | QQYSDDPT |
| | HCDR1 - SEQ ID NO: 16 |
| | SYAIS |
| | HCDR2 - SEQ ID NO: 17 |
| | SIIPWFGTTNYAQKFQG |
| | HCDR3 - SEQ ID NO: 18 |
| | DSEYYFDH |
| | VL - SEQ ID NO: 19 |
| | DIQMTQSPSSVSASVGDRVTITCRASQGISNWLNWYQQKPGKA PKLLIYAASSLQSGVPSRFSGSGSGTDFTLTISSLQPEDFATYYC QQYSDDPTFGQGTKVEIK |
| | VH - SEQ ID NO: 20 |
| | QVQLVQSGAEVKKPGSSVKVSCKASGGTFSSYAISWVRQAPGQ GLEWMGSIIPWFGTTNYAQKFQGRVTITADESTSTAYMELSSLR SEDTAVYYCARDSEYYFDHWGQGTLVTVSS |
| | LC - SEQ ID NO: 21 |
| | DIQMTQSPSSVSASVGDRVTITCRASQGISNWLNWYQQKPGKA PKLLIYAASSLQSGVPSRFSGSGSGTDFTLTISSLQPEDFATYYC QQYSDDPTFGQGTKVEIKGQPKAAPSVTLFPPSSEELQANKATL VCLISDFYPGAVTVAWKADSSPVKAGVETTTPSKQSNNKYAAS SYLSLTPEQWKSHRSYSCQVTHEGSTVEKTVAPTECS |
| | HC - SEQ ID NO: 23 |
| | QVQLVQSGAEVKKPGSSVKVSCKASGGTFSSYAISWVRQAPGQ GLEWMGSIIPWFGTTNYAQKFQGRVTITADESTSTAYMELSSLR SEDTAVYYCARDSEYYFDHWGQGTLVTVSSASTKGPSVFPLAP SSKSTSGGTAALGCLVKDYFPEPVTVSWNSGALTSGVHTFPAV LQSSGLYSLSSVVTVPSSSLGTQTYICNVNHKPSNTKVDKKVEP KSCDKTHTCPPCPAPELLGGPSVFLFPPKPKDTLMISRTPEVTCV VVDVSHEDPEVKFNWYVDGVEVHNAKTKPREEQYNSTYRVV SVLTVLHQDWLNGKEYKCKVSNKALPAPIEKTISKAKGQPREP QVYTLPPSRDELTKNQVSLTCLVKGFYPSDIAVEWESNGQPEN NYKTTPPVLDSDGSFFLYSKLTVDKSRWQQGNVFSCSVMHEAL HNHYTQKSLSLSPGK |

In certain embodiments, the detection agent, such as detection antibody, is labeled with a detectable label, preferably, the label is an enzyme or biotin. In a further embodiment, the detection agent is labeled with a marker such as biotin for interacting with a labelled enzyme such as streptavidin conjugated horse radish peroxidase, which works on tetramethylbenzidine in the presence of hydrogen peroxide to generate color signals for determination the amount of first detection agent.

As used herein, the terms "wash", "wash step", or "washing" refer to a process used to separate the total IL-17 (including both free IL-17 and modulator-bound IL-17), which are unbound to the capture antibody, from the mixture in step (i). The solution used for washing is generally a buffer ("washing buffer"). In some embodiments, the washing buffer contains low concentrations of detergents. In some embodiments, the washing buffer is a 10 mM phosphate buffer at a pH of about 7.4 comprising 150 mM NaCl and 0.05% Tween 20. The pH of the washing buffer is preferably in a range of about 6 to about 9. In some embodiments the pH is about 7.0. The wash step may be performed once or multiple times (e.g., one time, two times, three times, four times, five times, or six times). In a preferred embodiment, the wash step is performed three to six times.

In certain embodiments, the wash step is performed before contacting the first mixture from step (i) with the first detection agent in step (ii).

In certain embodiments, no wash step is performed before contacting the first mixture from step (i) with the first detection agent in step (ii).

As used herein, the term "LM modulator" or "LM modulator of IL-17" refers to any large molecule, including an antibody, or an antigen-binding fragment thereof, or a protein comprising an antigen binding fragment thereof, that can bind to IL-17 and have a molecular weight above about 1500 daltons, or preferably has a molecular weight ranging from about 2,000 daltons to about 250,000 daltons, or from about 5000 daltons to about 160,000 daltons, or from about 15,000 daltons to about 250,000 daltons, or from about 100,000 daltons to about 200,000 daltons, or from about 125,000 daltons to about 175,000 daltons.

The LM modulator used herein is an antibody that specifically binds to IL-17. Methods according to embodiments of the application can be used to measure total IL-17 in the presence of LM modulatorssecukinumab, mAb7024, mAb732, or binding fragments thereof.

Secukinumab (Caligor Coghlan, Secaucus, NJ), also disclosed in WO 2006/013107 (also published as US20090280131), is a recombinant high-affinity fully human monoclonal anti-human Interleukin-17A antibody of the IgG1/κ-class. Secukinumab has a molecular mass of approximately 151 kDa; both heavy chains of secukinumab contain oligosaccharide chains. Secukinumab binds to human IL-17A and neutralizes the bioactivity of this cytokine. Secukinumab has a very high affinity for IL-17, i.e., a KD of about 100-200 pM and an IC50 of about 0.4 nM for in vitro neutralization of the biological activity of 0.67 nM human IL-17A.

mAb7024 and mAb732 are antibodies of IL-17 are from Janssen Biotech, Inc. and disclosed in U.S. Patent. No. 8,519,107. Detailed sequence information for mAb7024 and mAb732 are listed in Table 1. mAb7024 has a molecular weight of about 146 kDa. mAb732 has a molecular weight of about 150 kDa.

In certain embodiments, the IL-17 is a human IL-17.

In certain embodiments, the sample is a biological sample obtained from a human subject under an *ex vivo* or *in vivo* treatment with a small molecule modulator of a human IL-17.

In certain embodiments, the biological sample is selected from the group consisting of a sample prepared from cells, tissues, or a serum, plasma or another biological fluid sample.

In general, commercially available kits (e.g., ELISA kits) only detect free IL-17, which is, due to its low level, rarely detectable in healthy subjects when administered with anti-IL-17 mAb. However, the method(s) described herein detect total IL-17 (including free and LM-bound IL-17), which provide measurable amounts of IL-17 that are above lower limit of detection. As a result, the claimed method(s) provide the quantitative evidence for target engagement by LM modulator(s). Moreover, analysis of clinical serum samples has shown dose- and exposure-dependent increases of IL-17 due to increases in half-life of IL-17/LM complex (Peng K. *et al*.), supporting the concept of measuring total IL-17 for target engagement assessment. Furthermore, the inventive assay described herein measures total IL-17 in the presence of LM modulators independent of the presence of disease and can be applied to healthy subjects or patients.

Additionally, as discussed above, even though Peng *et al.* developed an assay that measured target engagement specifically for an anti-IL-17 antibody, MCAF5352A, the method demonstrates utility with only a single antibody. The inventive method described herein, however, can be used to measure target engagement for a larger number of LM modulators. This target engagement readout and pharmacokinetics (PK) of the test molecule can be used in modeling to determine the optimal dose to achieve efficacy and minimize signals in safety studies.

In addition, the inventive method(s) described herein can be used in combination with commercially available assays that measure free IL-17 in the presence of LM modulators to determine the target engagement of LM modulators. Specifically, in samples comprising IL-17 and LM modulators, by subtracting the level of free IL-17 (determined using commercially available assays) from the level of total IL-17 (determined using the inventive capture antibody and detection agent pair disclosed herein), the target engagement extent of the LM modulators can be assessed.

The present application yet further provides a kit for determining an amount of total IL-17 in a sample comprising the IL-17 and a LM modulator, the kit comprising:
i. a capture antibody, wherein upon contact with the sample, the capture antibody forms a first complex comprising the capture antibody and the IL-17 unbound to the LM modulator, and a second complex comprising the capture antibody and the IL-17 bound to the LM modulator, respectively; and
ii. a detection agent (such as first detection antibody), wherein upon contacting with the first complex and the second complex, the detection agent forms a third complex comprising the capture antibody, the IL-17 unbound to the LM modulator and the detection agent, and a fourth complex comprising the capture antibody, the IL-17 bound to the LM modulator and the detection agent,
wherein the capture antibody is anti-IL-17 antibody AF-317 and the detection agent is mAb4538 and the large molecule (LM) modulator is selected from the group consisting of secukinumab, mAb7024, and mAb732 and the capture antibody is capable of forming a complex comprising the capture antibody and the IL-17 bound to any of secukinumab, mAb7024, and mAb732.

In some embodiments, the kit further comprises one or more reagents for detecting the detection agent.

### EXAMPLES

### Example 1

### Materials

The capture antibody/detection agent pairs used in the examples are listed in Table 2.

**Table 2**

| **Capture Antibody** | | | **Detection Agent** | | |
|---|---|---|---|---|---|
| **Company** | **CAT# or ID** | **Clone** | **Company** | **CAT# or ID** | **Clone** |
| R&D Systems Inc. | Capture antibody of DuoSet ELISA kit DY317 | Monoclonal Mouse IgG2B Clone # 41809 (Catalog Number: MAB317) | R&D Systems Inc. | Detection antibody of DuoSet ELISA kit DY317 | Antigen Affinity-purified Polyclonal Goat IgG (Catalog Number: BAF317) |
| R&D Systems Inc. | AF-317-NA | Antigen Affinity-purified Polyclonal Goat IgG | Janssen Biotech, Inc. | mAb4538 | N/A |

### Methods

*Screening of capture antibody*/*detection agent pairs that can detect total IL-17 (free and LM modulator-bound IL-17) with ELISA:* Two-fold and 8-point serial dilutions of recombinant IL-17 were prepared in diluent starting from final concentration at 1 ng/ml, in duplicate. Secukinumab (150 mg/ml Solution for Injection; NDC: 00078-0639-98; Caligor OPCO, LLC) was prepared for final concentration of 10 µg/ml, 1 µg/ml, 0.1 µg/ml, and 0.01 µg/ml, and 0.001 µg/ml.

High binding 96-well plates were coated with 100 µL/well of 4 µg/ml capture antibody in PBS overnight at room temperature. Coated plates were washed 6 × 400 µL/well on plate washer (Zoom HT Microplate Washer) and blocked with 300 µL/well blocker (1% BSA in PBS) at room temperature for 2.5 hours followed by 6 × 400 µL/well on plate washer.

IL-17 in samples after incubation with test molecules were measured in ELISA assay by adding 95 µL/well samples to the capture antibody coated plates. Plates were kept at room temperature for 30 minutes and then washed 6 × 400 µL/well on plate washer. 5 µL/well of detecting agent were added to a final of 500 ng/mL, kept at room temperature for another 30 minutes, followed by washing 6 × 400 µL/well on plate washer. Development of color for measurement was performed by adding streptavidin conjugated horseradish peroxidase at 100 µL/well and incubated for 20 minutes at room temperature in the dark, followed by 6 × 400 µL/well on plate washer, and then addition of 100 µL/well of substrate solution, incubated for 20 minutes at room temperature in the dark. Reaction was then stopped by addition of 50 µL/well of stop solution and plates were measured in plate reader at 450-540 nm.

Optical density (OD) readout from plate reader (Molecular Devices Spectra Max 340fPC) were analyzed with Softmax Pro6.3 and GraphPad Prism, and plotted as dose response curve of OD values vs IL-17 concentrations.

### Results

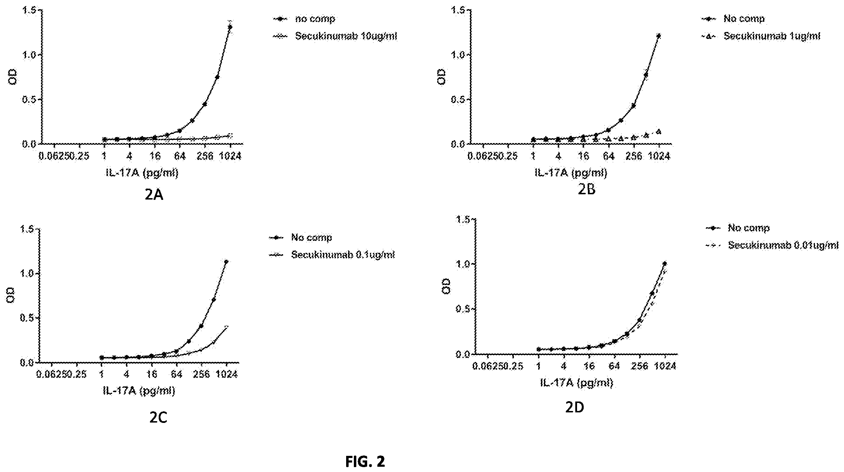

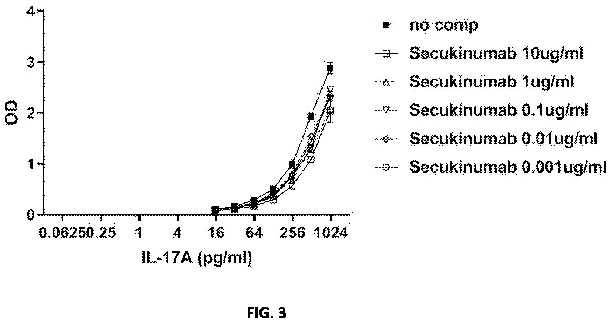

*Detection of free or total human IL-17 (**FIG. 2-3**):* FIG. 2A-2D shows the IL-17 level, using DuoSet DY317 ELISA kit in a sample containing 10 µg/ml, 1 µg/ml, 0.1 µg/ml, or 0.01 µg/ml of secukinumab, in comparison with the level in samples with no IL-17 LM modulators added. As shown herein, using DuoSet DY317 ELISA kit, only free IL-17 can be detected, but not modulator-bound IL-17. The level of free IL-17 was secukinumab concentration dependent, increasing level of free IL-17 with decreasing concentration of secukinumab. However, as shown in FIG. 3, when AF-317/mAb4538 was used as the capture antibody/detection agent pair, total IL-17 was detected in the presence of secukinumab, and IL-17 levels were independent of secukinumab concentrations

### Example 2

In this example, using similar ELISA assay as described in Example 1, the two (2) capture antibody/detection agent pairs (listed in Table 2) were tested using three (3) different LM modulators of IL-17, secukinumab (10 µg/ml), mAb7024 (10 µg/ml), and mAb732 (10 µg/ml).

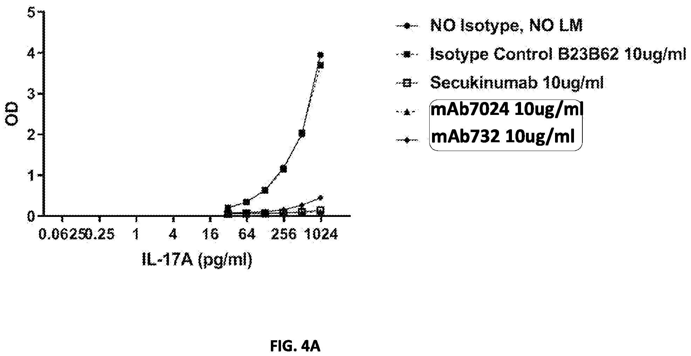

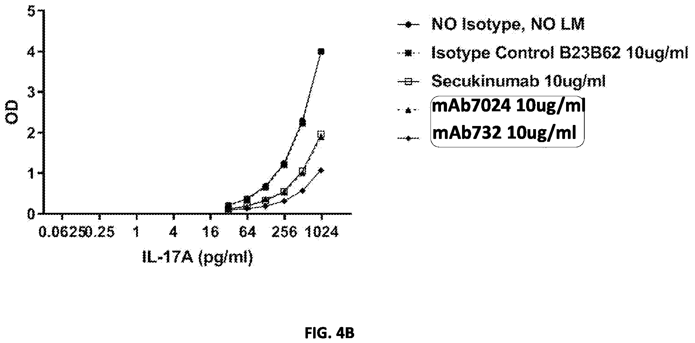

As shown in FIG. 4A, DuoSet DY317 ELISA kit detects free IL-17. In the presence of high concentration of LM modulators, IL-17 was bound to LM, therefore only zero to minimum level of free IL-17 was detected, and modulator-bound IL-17 was not detected. While when AF-317/mAb4538 was used as the capture antibody/detection agent pair, total IL-17 was detected, in a concentration dependent manner, in the presence of LM modulators (secukinumab, mAb7024, or mAb732).

## Claims

1. A method of determining a total amount of IL-17 (free IL-17 and LM modulator-bound IL-17) in a sample comprising an IL-17 and a large molecule (LM) modulator of IL-17, the method comprising:
i. contacting the sample with a capture antibody to form a mixture comprising a first complex comprising the capture antibody and the IL-17 unbound to the LM modulator, and a second complex comprising the capture antibody and the IL-17 bound to the LM modulator;
ii. contacting the mixture from step i) with a detection agent to thereby form a third complex comprising the capture antibody, the IL-17 unbound to the LM modulator and the detection agent, and a fourth complex comprising the capture antibody, the IL-17 bound to the LM modulator and the detection agent; and
iii. determining the amount of total IL-17 by measuring the amount of the detection agent in the third and fourth complexes,
wherein the capture antibody is anti-IL-17 antibody AF-317 and the detection agent is mAb4538 and the large molecule (LM) modulator is selected from the group consisting of secukinumab, mAb7024, and mAb732 and the capture antibody is capable of forming a complex comprising the capture antibody and the IL-17 bound to any of secukinumab, mAb7024, and mAb732.

2. The method of claim 1, wherein a wash step is performed before contacting the first mixture from step i) with the detection agent in step (ii).

3. The method of any one of claims 1-2, wherein the detection agent is labeled with a detectable label.

4. The method of claim 3, wherein the detectable label is an enzyme or biotin.

5. The method of any one of claims 1-4, wherein the sample is a biological sample obtained from a subject under an *ex vivo* or *in vivo* treatment with the LM modulator.

6. The method of claim 5, wherein, the subject is a mammal, preferably human.

7. The method of claim 5 or 6, wherein the biological sample is selected from the group consisting of a sample prepared from cells, tissues, or a serum, plasma or another biological fluid sample.

8. The method of any one of claims 1-7, which is an enzyme-linked immunosorbent assay (ELISA).

9. A kit for determining an amount of total IL-17 in a sample comprising an IL-17 and a LM modulator of IL-17, the kit comprising:
i. a capture antibody, wherein upon contact with the sample, the capture antibody forms a first complex comprising the capture antibody and the IL-17 unbound to the LM modulator, and a second complex comprising the capture antibody and the IL-17 bound to the LM modulator; and
ii. a detection agent, wherein upon contact with the first complex and the second complex, the detection agent, forms a third complex comprising the capture antibody, the IL-17 unbound to the LM modulator, and the detection agent, and a fourth complex comprising the capture antibody, the IL-17 bound to the LM modulator, and the detection agent,
wherein, the capture antibody is anti-IL-17 antibody AF-317 and the detection agent is mAb4538, the large molecule (LM) modulator is selected from the group consisting of secukinumab, mAb7024, and mAb732 and the capture antibody is capable of forming a complex comprising the capture antibody and the IL-17 bound to any of secukinumab, mAb7024, and mAb732.

10. The kit of claim 9, wherein the detection agent is labeled with a detectable label, more preferably, the label is an enzyme or biotin.

11. The kit of claim 9 or 10, which is for conducting ELISA.

## Patentansprüche

1. Verfahren zum Bestimmen einer Gesamtmenge an IL-17 (freies IL-17 und an LM-modulatorgebundenes IL-17) in einer Probe, umfassend ein IL-17 und einen Modulator großer Moleküle (LM-Modulator) von IL-17, das Verfahren umfassend:
i. Inkontaktbringen der Probe mit einem Fängerantikörper, um eine Mischung auszubilden, umfassend einen ersten Komplex, umfassend den Fängerantikörper und das IL-17, das an den LM-Modulator ungebunden ist, und einen zweiten Komplex, umfassend den Fängerantikörper und das IL-17, das an den LM-Modulator gebunden ist;
ii. Inkontaktbringen der Mischung aus Schritt i) mit einem Nachweismittel, wobei dadurch ein dritter Komplex ausgebildet wird, umfassend den Fängerantikörper, das IL-17, das an den LM-Modulator ungebunden ist, und das Nachweismittel, und einen vierten Komplex, umfassend den Fängerantikörper, das IL-17, das an den LM-Modulator gebunden ist und das Nachweismittel; und
iii. Bestimmen der Menge an Gesamt-IL-17 durch Messen der Menge des Nachweismittels in dem dritten und dem vierten Komplex,
wobei der Fängerantikörper Anti-IL-17-Antikörper AF-317 ist und das Nachweismittel mAb4538 ist und der Modulator großer Moleküle (LM-Modulator) aus der Gruppe ausgewählt ist, bestehend aus Secukinumab, mAb7024 und mAb732, und der Fängerantikörper in der Lage ist, einen Komplex auszubilden, umfassend den Fängerantikörper und das IL-17, das an ein beliebiges von Secukinumab, mAb7024 und mAb732 gebunden ist.

2. Verfahren nach Anspruch 1, wobei ein Waschschritt durchgeführt wird, bevor die erste Mischung aus Schritt i) mit dem Nachweismittel in Schritt (ii) in Kontakt gebracht wird.

3. Verfahren nach einem der Ansprüche 1 bis 2, wobei das Nachweismittel mit einer nachweisbaren Markierung markiert ist.

4. Verfahren nach Anspruch 3, wobei die nachweisbare Markierung ein Enzym oder ein Biotin ist.

5. Verfahren nach einem der Ansprüche 1 bis 4, wobei die Probe eine biologische Probe ist, die von einem Subjekt unter einer *ex-vivo-* oder *in-vivo-*Behandlung mit dem LM-Modulator erhalten wird.

6. Verfahren nach Anspruch 5, wobei das Subjekt ein Säugetier, vorzugsweise ein Mensch, ist.

7. Verfahren nach Anspruch 5 oder 6, wobei die biologische Probe aus der Gruppe ausgewählt ist, bestehend aus einer Probe, die aus Zellen, Geweben oder einer Serum-, Plasma- oder anderen biologischen Fluidprobe hergestellt wird.

8. Verfahren nach einem der Ansprüche 1 bis 7, das ein enzymgekoppelter Immunoabsorptionstest (ELISA) ist.

9. Kit zum Bestimmen der Menge an Gesamt-IL-17 in einer Probe, umfassend ein IL-17 und einen LM-Modulator von IL-17, das Kit umfassend:
i. einen Fängerantikörper, wobei, bei dem Inkontaktbringen mit der Probe, der Fängerantikörper einen ersten Komplex ausbildet, umfassend den Fängerantikörper und das IL-17, das an den LM-Modulator ungebunden ist, und einen zweiten Komplex, umfassend den Fängerantikörper und das IL-17, das an den LM-Modulator gebunden ist; und
ii. ein Nachweismittel, wobei, bei dem Inkontaktbringen mit dem ersten Komplex und dem zweiten Komplex, das Nachweismittel einen dritten Komplex ausbildet, umfassend den Fängerantikörper, das IL-17, das an den LM-Modulator ungebunden ist und das Nachweismittel, und einen vierten Komplex, umfassend den Fängerantikörper, das IL-17, das an den LM-Modulator gebunden ist, und das Nachweismittel,
wobei der Fängerantikörper Anti-IL-17-Antikörper AF-317 ist und das Nachweismittel mAb4538 ist, der Modulator großer Moleküle (LM-Modulator) aus der Gruppe ausgewählt ist, bestehend aus Secukinumab, mAb7024 und mAb732, und der Fängerantikörper in der Lage ist, einen Komplex auszubilden, umfassend den Fängerantikörper und das IL-17, das an ein beliebiges von Secukinumab, mAb7024 oder mAb732 gebunden ist.

10. Kit nach Anspruch 9, wobei das Nachweismittel mit einer nachweisbaren Markierung markiert ist, mehr bevorzugt, wobei die Markierung ein Enzym oder ein Biotin ist.

11. Kit nach Anspruch 9 oder 10, das zum Durchführen von ELISA dient.

## Revendications

1. Procédé de détermination d'une quantité totale d'IL-17 (IL-17 libre et IL-17 lié à un modulateur LM) dans un échantillon comprenant un IL-17 et un modulateur à grandes molécules (LM) d'IL-17, le procédé comprenant :
i. la mise en contact de l'échantillon avec un anticorps de capture pour former un mélange comprenant un premier complexe comprenant l'anticorps de capture et l'IL-17 non lié au modulateur LM, et un deuxième complexe comprenant l'anticorps de capture et l'IL-17 lié au modulateur LM ;
ii. la mise en contact du mélange de l'étape i) avec un agent de détection pour former ainsi un troisième complexe comprenant l'anticorps de capture, l'IL-17 non lié au modulateur LM et l'agent de détection, et un quatrième complexe comprenant l'anticorps de capture, l'IL-17 lié au modulateur LM et l'agent de détection ; et
iii. la détermination de la quantité d'IL-17 totale en mesurant la quantité de l'agent de détection dans les troisième et quatrième complexes,
dans lequel l'anticorps de capture est l'anticorps anti-IL-17 AF-317, l'agent de détection est le mAb4538 et le modulateur à grandes molécules (LM) est choisi dans le groupe constitué de secukinumab, mAb7024 et mAb732, et l'anticorps de capture est capable de former un complexe comprenant l'anticorps de capture et l'IL-17 lié à l'un quelconque de secukinumab, mAb7024 et mAb732.

2. Procédé selon la revendication 1, dans lequel une étape de lavage est réalisée avant de mettre en contact le premier mélange provenant de l'étape i) avec l'agent de détection dans l'étape ii).

3. Procédé selon l'une quelconque des revendications 1 à 2, dans lequel l'agent de détection est marqué d'un marqueur détectable.

4. Procédé selon la revendication 3, dans lequel le marqueur détectable est une enzyme ou de la biotine.

5. Procédé selon l'une quelconque des revendications 1 à 4, dans lequel l'échantillon est un échantillon biologique obtenu à partir d'un sujet soumis à un traitement ex *vivo* ou *in vivo* avec le modulateur LM.

6. Procédé selon la revendication 5, dans lequel le sujet est un mammifère, de préférence humain.

7. Procédé selon la revendication 5 ou 6, dans lequel l'échantillon biologique est choisi dans le groupe constitué d'un échantillon préparé à partir de cellules, de tissus, ou d'un échantillon de sérum, de plasma ou d'un autre fluide biologique.

8. Procédé selon l'une quelconque des revendications 1 à 7, qui est une technique d'immunoadsorption par enzyme liée (ELISA).

9. Kit permettant de déterminer une quantité d'IL-17 totale dans un échantillon comprenant un IL-17 et un modulateur LM d'IL-17, le kit comprenant :
i. un anticorps de capture, dans lequel, au contact de l'échantillon, l'anticorps de capture forme un premier complexe comprenant l'anticorps de capture et l'IL-17 non lié au modulateur LM, et un deuxième complexe comprenant l'anticorps de capture et l'IL-17 lié au modulateur LM ; et
ii. un agent de détection, dans lequel, au contact du premier complexe et du deuxième complexe, l'agent de détection forme un troisième complexe comprenant l'anticorps de capture, l'IL-17 non lié au modulateur LM et l'agent de détection, et un quatrième complexe comprenant l'anticorps de capture, l'IL-17 lié au modulateur LM et l'agent de détection,
dans lequel l'anticorps de capture est l'anticorps anti-IL-17 AF-317 et l'agent de détection est le mAb4538, le modulateur à grandes molécules (LM) est choisi dans le groupe constitué de secukinumab, mAb7024 et mAb732 et l'anticorps de capture est capable de former un complexe comprenant l'anticorps de capture et l'IL-17 lié à l'un quelconque de secukinumab, mAb7024 et mAb732.

10. Kit selon la revendication 9, dans lequel l'agent de détection est marqué avec un marqueur détectable, plus préférablement, le marqueur est une enzyme ou de la biotine.

11. Kit selon la revendication 9 ou 10, qui est destiné à la réalisation de la méthode ELISA.
